# EUROPEAN PATENT SPECIFICATION

(11) **EP 0 461 266 B1**
(45) Date of publication and mention of the grant of the patent: **25.02.1998**
(21) Application number: 91900951.4
(22) Date of filing: 25.12.1990
(51) Int. Cl.: C07F 7/22

(54) **PROCESS FOR PURIFYING ORGANOTIN (IV) POLYHALIDE**
VERFAHREN ZUR REINIGUNG VON ORGANOZINN(IV)-POLYHALOGENIDEN
PROCEDE DE PURIFICATION DE POLYHALOGENURE D'ORGANOTINE (IV)

(30) Priority: 28.12.1989 JP 3411/88
(43) Date of publication of application: 18.12.1991
(73) Proprietor: KORIYAMA KASEI CO., LTD., Tokyo 103 (JP)
(72) Inventor: SUZUKI, Yasuyuki c/o Koriyama Kasei Co. Ltd., Tokyo 103 (JP); WATANABE, Yuji c/o Koriyama Kasei Co. Ltd., Tokyo 103 (JP); FUJITA, Toshinao c/o Koriyama Kasei Co. Ltd., Tokyo 103 (JP); DEN, Naruo c/o Koriyama Kasei Co. Ltd., Tokyo 103 (JP)
(74) Representative: Behrens, Dieter, Dr.-Ing.
(86) International application number: PCT/JP90/01687
(87) International publication number: WO 91/09862

(56) References cited:
- JP-B- 5 146 092
- JP-B- 5 241 246
- JP-B- 5 921 873
- JP-B- 5 921 874
- US-A- 3 557 172
- US-A- 3 872 143
- CHEMICAL ABSTRACTS, vol. 98, 1983, Columbus, Ohio, US, abstract no. 54213p

## Description

This invention is concerned with a refining process of organic tin polyhalides which are useful as starting material of stabilizer for polyvinylpolymers, polymerization catalyst to polyurethane and builder of coated film layer of tin oxide on glass, ceramics or metals.

Aluminum Process in which stannic chloride reacts with alkyl aluminum, Grignard Process in which Grignard reagent reacts with stannic chloride and Direct Process in which alkyliodide directly reacts with metallic tin have been known as industrial production method of organic tin [IV] polyhalide. In both Alkyl Aluminum and Grignard Processes the corresponding chlorides are formed as an intermediate, on the other hand in Direct Process the corresponding iodide is formed as an intermediate.

For example, dialkyl tin (IV) dichloride, one of the polyhalide can be synthesized by Alkyl Aluminum or Grignard process via tetraalkyl tin (IV) as an intermediate, which is disproportionately reacted with equimolecular stannic chloride. Dialkyl tin(IV) diiodide can be synthesized by the Direct Method in which alkyl iodide and metallic tin are reacted with metallic magnesium and alcohol as catalyzer. However, the dialkyl tin (IV) dihalide synthesized by these methods always contains several % of trialkyl tin (IV) monohalide and monoalkyl tin (IV) trihalide as by-products.

It is desirable to eliminate organic tin (IV) monohalide from its polyhalide because it is well known that organic tin(IV) monohalides are commonly higher toxic than its polyhalides.

It may be possible to reduce the organic tin (IV) monohalide contained in organic tin (IV) polyhalide by addition and heating treatment with excess anhydrous stannic chloride for the monohalide and, as a catalyzer, aluminum chloride. However, this process is very expensive and difficult, because of its unselectivity of the reaction and disposal treatment of hydroxides formed from stannic chlorice involved in the main reaction and from aluminum chloride. Additionally, the monohalide is difficult to eliminate from monoalkyl tin (IV) polyhalide by distillation because the boiling point is too close in each other. Never method has been reported to eliminate economically a small content of trialkyl tin (IV) monohalide from monoalkyl tin (IV) trihalide and dialkyl tin (IV) dihalide which are synthesized in nearly same way.

Although a decomposition reaction from organic tin (IV) monohalide to the polyhalide by hydrochloric acid or chlorine was described in (Metal-Organic Compound, item 208 or Chemical Review Vol. 60 item 483), no example to eliminate organic tin (IV) monohalide in the polyhalide is demonstrated.

JP-B-59021873 discloses a process comprising (i) adding a dilute aqueous solution of an inorganic acid to the mixture of monoalkyl tin trihalide and one or more compounds selected from the group consisting of tetraalkyl tin, trialkyl tin monohalide, dialkyl tin dihalide and tin tretrahalide in a solvent, (ii) seperating the water phase and (iii) adding a solvent to the water phase to extract the monoalkyl tin trihalide out of the water phase into the organic solvent phase.

This invention concerns a process for refining organig tin(IV) polyhalide characterized in that organic tin(IV) polyhalide which contains organic tin(IV) monohalide is treated with hydrochloric acid, hydrogen chloride or chlorine at a temperature of 100 to 250°C, wherein the hydrochloric acid, hydrogen chloride or chlorine reacts with the organic tin(IV) monohalide.

Furthermore, the reaction efficiency can be remarkably improved by an addition of Lewis acid.

Equations of the reaction between organic tin (IV) monohalide and hydrochloric acid, hydrogen chloride or chlorine in the invention are as follows: ${\text{1) R}}_{\text{3}} {\text{SnX+HCl → R}}_{\text{2}} \text{SnXCl+RH}$${\text{2) R}}_{\text{3}} {\text{SnX+Cl}}_{\text{2}} {\text{→ R}}_{\text{2}} \text{SnXCl+RCl}$(wherein, R=alkyl or phenyl. X=chlorine, bromine or iodine)

Organic tin(IV)dihalides and organic tin(IV)trihalides are involved in organictin (IV) polyhalides in the invention.

Dibutyl tin (IV) dichloride, dibutyl tin (IV) diiodide, di-butyl tin (IV) dibromide, dioctyl tin (IV) dichloride, dioctyltin (IV) diiodide, dioctyl tin (IV) dibromide, diphenyl tin (IV)dichloride and diphenyl tin (IV) diiodide can be given as the examples of organic tin (IV) dihalides.

Butyl tin (IV) trichloride, butyl tin (IV) triiodide, butyltin (IV) bromide, octyl tin (IV) trichloride and octyl tin (IV)triiodide can be also given as the examples of organic tin (IV)trihalides. Organic tin (IV) monohalide in the invention is defined as the corresponding monohalide to each dihalides or trihalides previously given, then, tributyl tin (IV) monochloride , tributyl tin(IV) monoiodide, tributyl tin(IV) monobromide , trioctyl tin(IV) monochloride, trioctyl tin (IV) monoiodide, trioctyl tin (IV) monobromide, triphenyl tin (IV)monochloride and triphenyl tin (IV) monoiodide can be given as the examples.

Concentration of hydrochloric acid used in the treatment is higher than 20 %, preferably about 35 % is desirable. Either dried or wet hydrogen chloride gas or chlorine is also allowed to be profitably applied.

Proper condition of treatment temperature depends from the kind of organic tin(IV) polyhalide, the higher temperature the higher is the efficiency, but due to undesirable side reaction such as organic tin (IV) dihalide decomposes to organic tin(IV)trihalide occurring beyond 250 °C, it is favorably adaptable to keep between 100-200 °C. Quantity of reacted hydrochloric acid, hydrogen chloride or chlorine depends upon a kind of treated organic tin(IV) polyhalides, about 1-100 times on the molar basis of the organic tin (IV) monohalideis estimated as reasonable ratio, desirable 20-50 times molar.

Most of hydrochloric acid, hydrogen chloride or chlorine can be recovered, so so its net quantity consumed through the reaction is very slight.

It is allowable for the reaction procedure that organic tin(IV) polyhalide mixed already with hydrochloric acid is slowly heated under stirring condition or the latter is dropped into the former already heated. In this case higher contact efficiency of the stirring is desirable. In the reaction with hydrogen chloride or chlorine, it is introduced as a gas into already heated organic tin(IV) polyhalide which is to be treated. In this case the possible as higher contact of the gas with the reacting solution is also desirable, therefore, location at where the gas is introduced is more desirable to be inside of liquid organic tin (IV)polyhalide than its surface at where is even permitted to be blown. Treated time is desirable for 2 to 3 hours, 1 to 2 hours and 10minutes to 1 hour in the case of hydrochloric acid, hydrogenchloride or chlorine, respectively. The content of organic tin(IV) monohalide in the polyhalides is reduced to be less than 10ppm by this process.

A catalyzer, is not always necessary in this process, but which is effectively accelerated by a slight addition of Lewis acid. Most of all Lewis acid widely known are useful, for example AlCl₃, AlBr₃, AlI₃, GaBr₃, GaCl₃, FeCl₃, SbCl₅, SnCl₄, SnI₄ ,ZrCl₄, BCl₃, BF₃, BCl₃, and ZnCl₃, the most excellent of these is AlCl₃ because of lower expense and higher effect.

In addition to this process the refined organic tin(IV) polyhalide is successively hydrolyzed to organic tin(IV) oxide, which is further less contaminated by organic tin(IV) monohalide. The hydrolysis is carried out according to a method, for example, described in Japanese Patent No. Sho 61-291592 (1986).

### [Best Mode for Carrying out the invention]

The invention is concretely explained with giving examples as follows, but the scope of the invention is never limited by the following examples: (Reference Example 1). Synthesis of dibutyl tin diiodide

In a flask (300 ml) equipped with stirrer, reflux condenser and thermometer, 41.5 g of tin foil, 200 g of butyl iodide, 3g of butanol and 0.15 g magnesium foil was loaded and are re-fluxed on a mantel heater for 2 hours. After some tin foil reacted and was consumed, the refluxing reaction was further continued for 4 hours with stirring to disappear the tin foil.

Excess butyl iodide was recovered by vacuum distillation and residual solution was sufficiently washed by hydrochloric acid at room temperature, thus 168 g of dibutyl tin diiodide was obtained as pale yellowish clear liquid, which composition was analyzed by chromatography (abbreviated as GC, hereafter). The analytical result showed that it was composed of 4.2 % of monobutyl tin(IV) triiodide, 92.4 % of dibutyl tin (IV) diiodide, 2.3 % of tributyltin (IV) monoiodide and 1.1 % of others.

### (Example 1)

In a similar flask (300 ml) as Reference Example 1, 50 g of dibutyl tin diiodide obtained in the Reference Example 1 and 10 g of 35 % hydrochloric acid was put and slowly heated to refluxing temperature (about 120°C). From a dropping funnel, 30 g of 35 % hydrochloric acid was dropped into the flask during 30 minutes and water phase was removed. Just after the reacting solution was reached at refluxing temperature an evolution of hydrogen chloride from top of the reflux condenser was observed and it was recovered. Additionally, 30 g of35 % of hydrochloric acid was dropped into the flask and this procedure was repeated 5 times. The dropped total amount of hydrochloric acid was 150 g.

After the reaction the mixture was cooled at room temperature and discarded water phase by a separatory funnel. Powdery 24.9 g of dibutyl tin oxide was obtained by hydrolysis of the treated dibutyl tin diiodide according to a method described in Example 1 of Japanese Patent No. Sho 61-291592 (1986). Content of tributyl tin compounds in the obtained substance was analyzed by gas chromatography and was less than detectable limit.

### (Example 2)

In a similar apparatus as described Example 1, equipped with a distillation condenser instead of reflux one, 50 g of dibutyltin (IV) diiodide obtained in the Reference Example 1 was heated at 150°C and 30 g of 35 % hydrochloric acid was slowly added by dropping at 150-160 °C. As soon as the dropping began, the reaction was proceeded and water phase boiled, then was distilled out. After the dropping addition was completed the reaction mixture was cooled at room temperature and analyzed by GC. It was shown by GC analysis that the resulted mixture was composed of 4.9 % of monobutyl tin(IV) triiodide, 94.0 % of dibutyl tin (IV) diiodide, 0.6 % of tributyl tin (IV) monoiodide and 0.5 % of others.

Through a inserted glass tube inside the reaction mixture in the apparatus, hydrogen chloride was blown into at 150 °C for 60 minutes. The treated dibutyl tin (IV) diiodide was hydrolyzed as described Example 1. It was shown by GC analysis that tributyl tin compound in the obtained dibutyl tin(IV)diiodide was less than detectable limit.

### (Example 3)

In a similar apparatus as described in Reference Example 1, equipped with a blowing tube for gas, 50 g of dibutyl tin (IV)diiodide obtained in the Reference Example 1 was heated at 150°C under stirring condition. Through the inserted glass tube inside the reaction mixture in the apparatus, hydrogen chloride was blown into at the rate of 30 ml/min. keeping at the same temperature. The reaction was continued for 120 minutes with blowing the gas. When evolution of butane gas with unreacted hydrogen chloride was observed from top of the condenser,the reaction was discontinued. After cooling the reaction mixture at room temperature, it was hydrolyzed as described in Example 1. It was shown by GC analysis that tributyl tin compound in the obtained dibutyl tin (IV) diiodide was less than detectable limit.

### (Reference Example 2) Dibutyl tin (IV) dichloride

In a similar apparatus as described in Reference Example 1, 67 g of tetrabutyl tin (IV), 50 g of anhydrous stannic chloride and 3 g of anhydrous aluminum chloride were reacted at 140 °C for 3 hours. After cooling at room temperature, 100 ml of 1,1,1-trichloroethane was added and the reaction mixture was washed by two portions of 50 ml of 5 % hydrochloric acid and water phase was separated to discard. After the trichloroethane was eliminated by evaporation, then dibutyl tin(IV) dichloride was obtained as pale yellowish liquid, which was composed of 2.1 % of monobutyl tin(IV) trichloride, 94.2 % of dibutyl tin(IV) di-chloride, 3.4 % of tributyl tin(IV) monochloride and 0.3 % of others according to GC analysis.

### (Example 4)

In a similar apparatus as described in Reference Example 1, 50 g of liquid dibutyl tin (IV) dichloride obtained in Reference Example 2 and 40 g of 35 % hydrochloric acid were heated to reflux temperature and kept at the temperature for 30 minutes. After water phase was removed from the reaction mixture , which was refluxed with 40 g of 35 % hydrochloric acid. The procedures were repeated 5 times. After terminating the reaction the mixture was cooled at room temperature and the water phase was removed. Powdery 41.5 g of dibutyl tin oxide was obtained by hydrolysis of the resulted substance according to a method described in Example 3 of Japanese Patent No. Sho 61-291592 (1986).
Content of tributyl tin compound in the oxide was analyzed by gas chromatography and was less than detectable limit.

### (Example 5)

In a similar apparatus as described in Reference Example 2 equipped with a blowing tube for gas, 50 g of dibutyl tin (IV)dichloride obtained in the Reference Example 2 was heated at 150 °C under stirring condition. Through the inserted glass tube inside the reaction mixture in the apparatus, hydrogen chloride was blown into at the rate of 30 ml/min. keeping at the same temperature for 2 hours. After cooling the reaction mixture at room temperature, it was hydrolyzed as described in Example 4. It was shown by GC analysis that tributyl tin compound in the obtained dibutyl tin (IV) diiodide was less than detectable limit.

### (Example 6)

In a similar apparatus as described in Reference Example 2, equipped with a blowing tube for gas, 50 g of dibutyl tin (IV)dichloride obtained in the Reference Example 2 and anhydrous aluminum chloride were heated at 150°C under stirring condition. Through the inserted glass tube inside the reaction mixture in the apparatus, hydrogen chloride was blown into at the rate of 30ml/min. keeping at the same temperature for 1 hour. After cooling the reaction mixture at room temperature. It was shown by GC analysis that tributyl tin compound in the obtained dibutyl tin(IV) dichloride was less than detectable limit.

### (Example 7)

In a similar apparatus as described in Reference Example 1, equipped with a blowing tube for gas, 50 g of monobutyl tin (IV)trichloride which is composed of 5.4 % of tributyl tin (IV)monochloride, 3.1 % of dibutyl tin (IV) dichloride, 91.2 % of monobutyl tin (IV) trichloride and 0.3 % of others, and 1.5 g of anhydrous aluminum chloride were heated at 150 °C under stirring condition. Through the inserted glass tube inside the reaction mixture in the apparatus, hydrogen chloride was blown into at the rate of 30 ml/min. keeping at the same temperature for 20 minutes. After the reaction, it was shown by GC analysis that the obtained substance was composed of 2.9 % of dibutyl tin (IV) dichloride, 96.9 % of monobutyl tin (IV) trichloride, less than detectable limit (10 ppm) of tributyl tin (IV) monochloride and 0.5 % of the others.

### (Example 8)

In a similar apparatus as described in Reference Example 2,equipped with a blowing tube for gas, 50 g of dibutyl tin (IV)dichloride obtained in the Reference Example 2 was heated at 150 °C under stirring condition. Through the inserted glass tube inside the reaction mixture in the apparatus, chlorine was blown into at the rate of 30 ml/min. keeping at the same temperature for 30 minutes. After cooling the reaction mixture at room temperature. It was shown by GC analysis that tributyl tin compound in the obtained dibutyl tin (IV) diiodide was less than detectable limit (10 ppm).

### [Industrial Applicability]

In organic tin(IV) polyhalides and organic tin(IV) oxides manufactured by formerly developed industrial process, organic tin (IV) monohalide has always contaminated in the products. According to the invention, the organic tin (IV) monohalide can be eliminated from the products so that it can not be detected in sensitivity of ordinary gas chromatography.

## Claims

1. A process for refining organic tin(IV) polyhalide characterized in that organic tin(IV) polyhalide which contains organic tin(IV) monohalide is treated with hydrochloric acid, hydrogen chloride or chlorine at a temperature of 100 to 250°C, wherein the hydrochloric acid, hydrogen chloride or chlorine reacts with the organic tin(IV) monohalide.

2. A process according to claim 1 wherein Lewis acid is applied as a catalyst.

## Patentansprüche

1. Verfahren zur Reinigung von organischem Zinn(IV)-Polyhalogenid, dadurch gekennzeichnet, daß das organische Zinn(IV)-Polyhalogenid, das organisches Zinn(IV)-Monohalogenid enthält, mit Salzsäure, Hydrogenchlorid oder Chlor bei einer Temperatur von 100 bis 250°C behandelt wird, wobei die Salzsäure, das Hydrogenchlorid oder das Chlor mit dem organischen Zinn(IV)-Monohalogenid reagiert.

2. Verfahren nach Anspruch 1, worin Lewis-Säure als Katalysator eingesetzt wird.

## Revendications

1. Procédé de purification de polyhalogénure d'étain(IV) organique, caractérisé en ce qu'on traite du polyhalogénure d'étain (IV) organique contenant du monohalogénure d'étain (IV) organique par de l'acide chlorhydrique, du chlorure d'hydrogène ou du chlore à une température de 100 à 250°C, l'acide chlorhydrique, le chlorure d'hydrogène ou le chlore réagissant avec le monohalogénure d'étain(IV) organique.

2. Procédé selon la revendication 1, où un acide de Lewis est utilisé comme catalyseur.
